Europäisches Patentamt

(19) European Patent Office

Office européen des brevets

(11) Publication number: **0 112 147**
**A2**

(12) **EUROPEAN PATENT APPLICATION**

(21) Application number: **83307545.0**

(22) Date of filing: **12.12.83**

(51) Int. Cl.³: **C 12 N 11/08**
**C 12 P 19/22**

(30) Priority: **14.12.82 GB 8235537**

(43) Date of publication of application:
**27.06.84 Bulletin 84/26**

(84) Designated Contracting States:
**AT BE DE FR IT NL SE**

(71) Applicant: **CPC INTERNATIONAL INC.**
**International Plaza P.O. Box 8000**
**Englewood Cliffs New Jersey 07632(US)**

(72) Inventor: **Stouffs, Robert H. M.**
**336 Blvd du Souverain**
**B-1160 Brussels(BE)**

(72) Inventor: **Walon, Raoul G.P.**
**Avenue d'Huldergem 25**
**B-1020 Brussels(BE)**

(74) Representative: **Pennant, Pyers et al,**
**5 Quality Court Chancery Lane**
**London WC2A 1HZ(GB)**

(54) **Continuous enzymatic process for producing maltose from starch and starch hydrolysates.**

(57) An immobilized beta-amylase supported on a phenolic resin of defined total surface area and porosity is useful in preparing a high maltose syrup in a continuous process from starch hydrolysate by continously contacting the starch hydrolysate with the immobilized beta-amylase.

EP 0 112 147 A2

CONTINUOUS ENZYMATIC PROCESS FOR PRODUCING MALTOSE
FROM STARCH AND STARCH HYDROLYSATES

## BACKGROUND OF THE INVENTION

### Field of the Invention

The present invention relates to an immobilised beta-amylase enzyme and to its use in a process for producing syrups having high maltose content.

### The Prior Art

Maltose and high maltose syrups are conventionally prepared from starch by saccharifying a suitable starch substrate with beta-amylase, with or without addition of pullulanase, as disclosed in U.S. Patent No. 3,565,765.

In recent years, the art of immobilizing enzymes has been developed to facilitate extremely efficient and economic enzyme use and permit the design of continuous enzymatic processes by placing the immobilized enzyme in a bed or column. The enzymatic reaction is then conducted by continuously passing or percolating the substrate through the bed or column.

Problems have been encountered in the development of continuous processes for maltose production utilizing immobilized beta-amylase. For example, if one tries to adapt the process disclosed and claimed in U.S. Patent No. 3,868,304 (which involves immobilizing glucose
isomerase by adsorption onto a porous alumina carrier
material and employing the thus immobilized enzyme to
isomerise glucose to fructose) to a continuous column
process for maltose production with beta-amylase adsorbed
onto the same type of carrier material, the beta-amylase
rapidly loses its activity. The process, therefore, is
far less economical than a conventional batch process
using non-immobilized beta-amylase.

One resolution of the stability problem is to chemically
couple the beta-amylase to the carrier. For example,
the beta-amylase can be chemically attached to a crosslinked copolymer of acrylamide - acrylic acid using a
water soluble carbodiimide. This and other chemical
coupling techniques are discussed in "Preparation of
Immobilized Two-Enzyme System, Beta-Amylase-Pullulanase,
to an Acrylic Copolymer for the Conversion of Starch
to Maltose. I. Preparation and Stability of Immobilized
Beta-Amylase" Biotechnology and Bioengineering, Vol.
XVI, pages 567-577 (1974), "II. Co-coupling of the
Enzymes and Use in a Packed Bed Column" Ibid pages
579-591. "III. Process Kinetic Studies on Continuous
Reactors", Ibid pages 1567-1587. Per Vretblad and Rolf
Axen "Preparation and Properties of an Immobilized Barley
Beta-Amylase" Biotechnology and Bioengineering , Vol. XV,
pages 783-794 (1973).

Even though chemical coupling is effective to protect the working life of beta-amylase and therefore permit continuous column saccharification processes to produce maltose, the chemical method of immobilization has drawbacks on an industrial scale due to the high cost. It involves expensive chemicals and complicated chemical reactions. Moreover, it is often difficult, expensive and sometimes impossible to regenerate and thereby recover the carrier material after the enzyme activity has been exhausted.

Industry generally prefers to immobilize enzymes onto porous adsorbent carriers as described in U.S. Patent No. 3,868,304. Immobilization or loading of enzyme onto such carriers is extremely simple, involving merely filling a column or other reaction vessel with the carrier and simply passing a solution of the enzyme through it. Regeneration involves such simple techniques as pyrolysis or passing chemicals through the column with little or no destruction of the carrier material. Unfortunately, for beta-amylase, this process has the disadvantage of very short column life.

As indicated above, it is known that beta-amylase is a fairly sensitive enzyme, and particularly in dilute solution, rapidly loses its activity. However, thiol

./.

containing compounds such as serum albumin, cysteine and glutathione have been found to be effective protective agents for dilute solutions of the enzyme.

In U.K. Patent Specification 1,543,094 a process is disclosed for immobilizing beta-amylase by physical adsorption onto a porous carrier material wherein the saccharification is conducted in the presence of organic material containing a protective thiol function. The organic material is selected from thiol containing amino acids, thiol containing natural polypeptides, thiol containing water insoluble synthetic polymers and mixtures thereof. When water-soluble thiol containing materials are used in accordance with this process, however, they end up in the final maltose syrup and generally have to be removed. Accordinly, the 1,543,094 patent recommends the use of a water insoluble synthetic polymer containing thiol functions.

A continuous system for immobilizing enzymes on macroporous or macroreticular carriers by a physical bonding of the enzymes onto the carrier is disclosed in Belgian Patent No. 862,765. The process utilizes both cationic and anionic resins and macroporous polymeric resins having no ionic behavior whatsoever. According to the reference, beta-amylase can be immobilized on Duolite ES-762 resin (a macroporous polymer having no ion exchange activity but high capacity as an enzyme adsorbent) available from Diamond Shamrock. The resin is rinsed with methanol followed by buffering to pH 5.4 with a 1% solution of $CH_3COONa/CH_3COOH$. Beta-amylase enzyme is immobilized on the carrier and perliter of cysteine is added to protect the enzyme.

In U.K. Patent No. 1,557,944, there is described in Example 24 the production of a maltose syrup by contacting a soluble starch solution with a beta-amylase supported on a phenolic resin modified with diethylaminoethylamine. The amount of maltose produced in this manner is reported to be 12 mg/ml of the effluent from the column of immobilized enzyme.

It has now been found, in accordance with the present invention, that phenolic exchange resins of defined porosity, such as Duolite ES-568 (available from Diamond Shamrock) can be used as carriers for immobilizing beta-amylase. The immobilized beta-amylase has a long effective life and can be used to produce syrups of high maltose content, namely, from 30 to over 55% maltose.

./.

## SUMMARY OF THE INVENTION

According to the invention an immobilized beta-amylase system for producing maltose syrup from starch hydrolysate comprises beta-amylase enzyme immobilized on a porous phenolic resin, characterized in that said resin has a total surface area of about 40 to about 200 square meters per gram in the range of pores above about 30$\overset{o}{A}$ of which at least 30% of the pores have a radius of about 30 to about 250$\overset{o}{A}$ .

A further aspect of the invention is a continuous process for converting starch hydrolysate to a maltose syrup characterized by continuously contacting a starch hydrolysate with an immobilized beta-amylase system.

Preferably the porous phenolic resin is an anion exchange type resin. Preferred resins are those in which of the pores having a radius of about 30 to about 250 A at least 50% have a radius of 50 to 150 $\overset{o}{A}$, and/or at least 20% have a radius of about 60 to about 90 $\overset{o}{A}$, and/or at least 20% have a radius of about 70 to about 80 $\overset{o}{A}$. As the pore sizes refer to the resin when in use, it is convenienct for the resin to have a water swelling capacity of less than 10% by volume.

./.

The Figure reproduced as an attachment to European Patent Application 81102183.1 (Publication number 0036 662) is a differential pore radius distribution curve of several carriers, including the carrier used in the system of this invention. In more detail, the porous phenolic resin carriers defined in the claims have a porosity profile similar to curve I in the Figure. This curve was obtained by measuring pore size and volume by mercury porosimetry, plotting an integral curve expressing the relation between the volume condensed in the pores and the radii, and differentiating the integral curve to obtain the more illustrative differential distribution curve which expresses the increment of pore volume corresponding to a differential increase in their radius. A detailed description of this measurement, together with a description of the measurement of surface area, is set forth in "Adsorption On Solids", V. Ponec, et al., Butterworths, 1974, pages 38-39, 540-545 and 552-555.

While any phenolic resin having the surface area and porosity defined herein can be used in the systems of this invention, it is preferred to employ a resin having an anion exchange capacity. A specific resin found to be outstanding is Duolite ES-568. This is a phenolformaldehyde resin available from Diamond Shamrock.

./.

## DETAILED DESCRIPTION OF THE INVENTION

Syrups produced in accordance with the present invention may have a maltose content of at least 30 percent to over about 55 percent by weight of the dry solid content of the syrup. (All percentages stated herein are by weight unless indicated otherwise.) They have numerous uses as flavoring components and additives for foodstuffs including soft drinks, confections, bread doughs, beer and infant and invalid foods. They are also used in printing compositions and other industrial applications.

The continuous process of the present invention is preferably conducted in a column. The resin is first loaded in the column. Beta-amylase is then immobilized on the resin. This is followed by passing starch hydrolysate through the column to produce high maltose syrups.

The resin is prepared for use in the present invention by loading it into a column and washing it with demineralized water to remove any entrapped air bubbles. It is then washed with an alkali such as ammonium hydroxide. Excess alkali is then washed out with demineralized water until a pH from about 4.5 to about 8.0, preferably from about 5.5 to about 7.0 is reached.

./.

The beta-amylase enzyme employed in the practice of the invention can be a plant or a bacterial beta-amylase. Particularly suitable beta-amylases are those derived from malt extract.

Beta-amylase is immobilized on the resin by circulating it in solutions through the column. After that, the resin is washed with demineralized water and the effluent is collected to determine bound enzyme.

Starch hydrolysate is then introduced into the column. Particularly suitable starch hydrolysates are enzyme-thinned corn maltodextrins having a dextrose equivalent (hereinafter "DE") from about 12 to about 20 and a dry substance (hereinafter "%d.s.") from about 30 to about 55.

Process pH is controlled at from about 5.5 to about 7.0 using a suitable alkali such as sodium hydroxide, sodium carbonate or ammonium hydroxide added to the substrate feed.

The products of the present invention are high in maltose content. Maltose concentrations from about 30 percent to over about 55 percent of the dry solid content are easily achieved as demonstrated by the following experimental results. Column lives in excess of 1500 hours are also commonly attained.

./.

## E X A M P L E S

### Example 1

Three columns having dimensions of 2.5 x 30 cm were packed with 100 ml of Duolite ES-568 resin. The resin was washed upflow with demineralized water at 60°C for one hour to remove any entrapped air bubbles. Then, the packed resin was washed downflow with 2% ammonium hydroxide at 25°C for 1 hour at a rate of 4 bed volumes per hour (hereinafter "BVH"). Next, the excess of alkali was washed out with demineralized water until pH 6.0 was reached in the effluent. Then, a beta-amylase solution was offered at an enzyme load of 34 units per ml carrier and recirculated for 24 hours at a flow rate of 4 BVH in order to immobilize the enzyme on the resin. After that, the resin was washed with demineralized water at a rate of 4 BVH for 1 hour and the effluent collected for bound enzyme determination. The amount of bound enzyme was then calculated using the equation :

$$\text{\% Bound enzyme}^{x} = 100 - \frac{(\text{Units/ml measured in effluent} \times 400 \times 100)}{\text{Total units of enzyme offered}}$$

Assuming one unit of beta-amylase to be the amount of enzyme activity that is required to produce from soluble starch one millimole of maltose per minute at pH 4.5 and 45°C, a

---

[x] % Bound enzyme = 100% - unbound enzyme. The unbound enzyme was determined from the activity of the 4 bed volumes of effluent collected in the final water wash. This activity is measured in units per ml effluent. In this case, 4 bed volumes through a 100 ml column represents 400 ml. Thus, total activity in the effluent is 400 x units/ml effluent. This is then divided by the total units of enzyme offered. The fraction is then multiplied by 100 to give % unbound enzyme.

binding of 98.4% was determined as the average for the various columns. This was based on 3400 total units of enzyme offered and 0.14 units/ml measured in the effluent.

Next, the columns were fed with solutions of 12 DE enzyme-thinned corn maltodextrin at 30%, 40% and 50% d.s. respectively. The maltodextrin feeds had been clarified but not demineralized. They contained about 150 ppm $SO_2$ and were adjusted to pH 6.0 with sodium hydroxide. The three columns were sweetened with their respective feeds for one hour at room temperature. The column temperatures were then raised to 50°C and the flow rates adjusted to produce a 55% maltose syrup (based on dry substance).

The initial BVH rates obtained at the various substrate dry substances were :

| % d.s. | Initial BVH |
|--------|-------------|
| 30 | 6.2 |
| 40 | 4.8 |
| 50 | 3.2 |

Under these conditions, at each dry substance, sugar content was analyzed. The syrup produced had the following composition :

| | |
|--------|-------------|
| Dextrose | 1.5% dry substance |
| Maltose | 54.5 - 55.5% dry substance |
| D. P. 3 | 15.2 - 15.5% dry substance |
| D. P. 4+ | 27.5 - 28.8% dry substance |

Example 2

To demonstrate that the system and process of this invention can be used for extensive periods of time, runs were made to determine the long term productivity of the immobilized enzyme preparation.

A column was loaded with beta-amylase according to the general procedure of Example 1. The beta-amylase solution was prepared from a commercially available concentrated malt extract, High Diastase 1500 from A.B.M. Chemicals Ltd.

The feed solution was a 20 DE demineralized cornbased maltodextrin at 40% d.s. to which calcium chloride was added at 150 ppm Ca++. The feed contained 150 ppm $SO_2$ as a stabilizer and was buffered to pH 6.0 with sodium hydroxide. The column was operated at 50°C and flow rate was adjusted during the process to ensure a conversion based on dry solid to 50% maltose.
The process conditions during this trial were :

|  |  |
|---|---|
| Initial BVH (50% maltose) | 5.3 |
| 1st Half life (Days) | 44.6 |
| 2nd Half life (Days) | 18.3 |
| Total life (Days) | 72(a) |
| Total bed volumes | 4987 |
| Productivity, kg d.s./liter carrier | 2339 |

(a) Total life represents the total operation time until 0.5 BVH.

./.

As can be seen, the process can be operated continuously for more than 2 months and 1 liter of carrier-enzyme conjugate produces over 2.3 tons of high maltose syrup solids at 40% d.s. and 50% maltose.


Example 3

To demonstrate that the process can be used at high dry substance, the general procedure of Examples 1 and 2 was followed but the column was fed with a 20 DE maltodextrin at 50% d.s. and calcium chloride at 200 ppm Ca++. Some 200 ppm $SO_2$ were added to the feed.

The results were :

| | |
|---|---|
| Initial BVH 50% maltose | 3.0 |
| 1st Half life (Days) | 23 |
| 2nd Half life (Days) | 45 |
| Total life (Days) | 86(a) |
| Total bed volumes | 2624 |
| Productivity, kg d.s./liter carrier | 1611 |

(a) Total life represents the total operation until 0.5 BVH.

In spite of the lower productivity obtained at 50% d.s., when compared to the 40% d.s. trial (Example 2), the column operation at 50% d.s. presents the following advantages :

    1)   savings in evaporation costs when using as feed a starch hydrolysate produced at 50% d.s.

2)   lower risk of microbial spoilage when working
     at 50% d.s.

3)   longer total lives (days) of beta-amylase
     columns when operated at 50% d.s.

Example 4

In this Example a carrier used in the system according
to the present invention was compared with other carriers
according to the general procedure of Examples 1 and 2.
The columns were fed with a 20 DE maltodextrin at 40% or
50% dissolved solids to which calcium chloride had been
added at a level of 150 ppm and sulphur dioxide also at
150 ppm.   The results are given in the following Table

| Carrier | ds | Initial BVH (50% maltose) | Total life[a] (days) |
|---|---|---|---|
| Duolite ES-568 | 40% | 5.3 | 72 days |
| Duolite ES-762 | 40% | 3.7 | 30 days[b] |
| Duolite ES-771 | 40% | 3.5 | 33 days |
| Akzo Gamma-Alumina (0.04 - 1.5 E) | 40% | 3.8 | 23 days |
| Corning Alumina | 40% | 4.9 | 26 days |
| Amberlite IRA-904 | 50% | 1.0 | 9 days |
| Lewatit MP-64 | 50% | 1.1 | 14 days |

(a) Total life represents the total operation time until
    0.5 BVH.

(b) Estimated total life.

./.

CLAIMS

1. An immobilized beta-amylase system for producing maltose syrup from starch hydrolysate comprising beta-amylase enzyme immobilized on a porous phenolic resin, characterized in that said resin has a total surface area of about 40 to about 200 square meters per gram in the range of pores above about $30\overset{o}{A}$ of which at least 30% of the pores have a radius of about 30 to about $250\overset{o}{A}$ .

2. The system of claim 1 characterized in that said porous phenolic resin is an anion exchange type resin.

3. The system of claims 1 or 2 characterised in that at least 50% of the pores having a radius of about 30 to about $250\overset{o}{A}$ have a radius of 50 to $150\overset{o}{A}$ .

4. The system of any one of claims 1-3 characterized in that at least about 20% of the pores having a radius of about 30 to about $250\overset{o}{A}$ have a radius of about 60 to about $90\overset{o}{A}$ .

5. The system of any one of claims 1-4 characterized in that at least about 20% of the pores having a radius of about 30 to about $250\overset{o}{A}$ have a radius of about 70 to $80\overset{o}{A}$ .

6.  A continuous process for converting starch hydrolysate
    to a maltose syrup characterized by continuously
    contacting a starch hydrolysate with an immobilized
    beta-amylase system according to any one of the
    preceding claims.

7.  The process of claim 6 characterized in that said
    starch hydrolysate has a DE from about 12 to about
    20 and a % d.s. from about 30 to about 55 and the
    syrup produced contains dissolved solids comprising
    at least 30% maltose.

8.  The process of claims 6 or 7 characterized in that
    said starch hydrolysate is maintained at a pH from
    about 5.5 to about 7.0 with an alkali selected from
    the group consisting of sodium hydroxide, sodium
    carbonate and ammonium hydroxide.

9.  The process of any one of claims 6, 7 or 8 characterized in that the immobilized beta-amylase enzyme
    has a pH from about 4.5 to about 8.0.